# EUROPEAN PATENT SPECIFICATION

(11) **EP 1 928 357 B1**
(45) Date of publication and mention of the grant of the patent: **10.11.2010**
(21) Application number: 06816076.1
(22) Date of filing: 28.09.2006
(51) Int. Cl.: A61F 2/24

(54) **SYSTEM AND METHOD FOR DELIVERING A MITRAL VALVE REPAIR DEVICE**
SYSTEM UND VERFAHREN ZUR ABGABE EINER MITRALKLAPPEN-REPARATURVORRICHTUNG
SYSTEME ET PROCEDE DE POSE D'UN DISPOSITIF DE REPARATION VALVULAIRE MITRALE

(30) Priority: 28.09.2005 US 238853
(43) Date of publication of application: 11.06.2008
(73) Proprietor: Edwards Lifesciences Corporation, Irvine, CA 92614 (US)
(72) Inventor: BOURANG, Henry, CA 92604 (US); PINTOR, Rafael, CA 92692 (US); FARIABI, Sepehr, CA 92657 (US); SOLEM, Jan, Otto, CH-8234 Stetten XX (CH); KIMBLAD, Per, Ola, S-22457 Lund XX (SE); HARNEK, Jan, S-217 73 Malmo XX (SE)
(74) Representative: Baker, Colin John
(86) International application number: PCT/US2006/038540
(87) International publication number: WO 2007/038786

(56) References cited:
- WO-A-99/47075
- WO-A2-2004/045463
- US-A- 6 126 685
- US-A1- 2003 078 465
- US-A1- 2004 215 312
- US-A1- 2005 177 228

## Description

### FIELD OF THE INVENTION

The present invention relates to a delivery system and method, and more particularly to a delivery system and method for delivering a mitral valve repair device.

### BACKGROUND

Heart valve regurgitation, or leakage from the outflow to the inflow side of a heart valve, is a condition that occurs when a heart valve fails to close properly. Regurgitation through the mitral valve is typically caused by changes in the geometric configurations of the left ventricle, papillary muscles and mitral annulus. Similarly, regurgitation through the tricuspid valve is typically caused by changes in the geometric configurations of the right ventricle, papillary muscles and tricuspid annulus. These geometric alterations result in incomplete coaptation of the valve leaflets during systole.

A variety of heart valve repair procedures have been proposed over the years for treating defective heart valves. With the use of current surgical techniques, it has been found that between 40% and 60% of regurgitant heart valves can be repaired, depending on the surgeon's experience and the anatomic conditions present. The advantages of heart valve repair over heart valve replacement are well documented. These advantages include better preservation of cardiac function and reduced risk of anticoagulant-related hemorrhage, thromboembolism and endocarditis.

In recent years, several new minimally invasive techniques have been introduced for repairing defective heart valves wherein surgery and cardiopulmonary by-pass are not required. Some of these techniques involve introducing an implant into the coronary sinus for remodeling the mitral annulus. The coronary sinus is a blood vessel that extends around a portion of the heart through the atrioventricular groove in close proximity to the posterior, lateral and medial aspects of the mitral annulus. Because of its position, the coronary sinns provides an ideal conduit for receiving an implant (i.e., endovascular device) configured to act on the mitral annulus.

As a result of the development of implants configured for insertion into the coronary sinus for repairing mitral valves, new systems and methods for delivering these implants have also been developed. For example, U.S. Patent No. 6,210,432 to Solem et al., describes a stabilizing instrument onto which an implant may be mounted using a locking device including a pair of spring blades and knobs. After the implant is placed in a desired location in a patient, a catheter may be used to release the implant from the stabilizing instrument. In another example, U.S. Patent No. 6,402,781 to Langberg et al. describes a deployment system including an introducer sheath and a pusher mechanism. The implant is contained within the introducer sheath during advancement into the coronary sinus. After reaching the desired location, the pusher mechanism is used to hold the implant in a fixed position while the introducer sheath is retracted. WO-A-2004/045463 discloses a medical device for treating a defective heart valve. The medical device comprises a distal anchoring member for disposing in a blood vessel, a proximal anchoring member for disposing in or at an entrance of the blood vessel and a telescoping assembly coupling at a first end to the distal anchoring member and at a second end to the proximal anchoring member.

Although a number of delivery systems have been proposed for delivering medical implants, it has bean found that existing delivery systems are not always adequate and are not well-suited for use with recently developed medical, implant technology. Accordingly, a need exists for a new and improved delivery system that is better configured for use with new medical implant technology, thereby improving the safety and effectiveness of the procedure. It is desirable that such a delivery system be shaped to facilitate percutaneous advancement through a patient's vasculature to the coronary sinus. It is also desirable that such a delivery system be configured to deliver and deploy a medical implant in a every predictable and secure manner. It is also desirable that such a delivery system be capable of deploying the implant at a precise location. It is also desirable that the delivery system be configured for easy pre-procedure and peri-procedure flushing of all of the delivery lumens as well as adequate purging of air bubbles trapped in the catheter system to minimize the potential for air embolization during use of the delivery system. The present invention addresses these needs.

### SUMMARY OF THE INVENTION

According to the present invention an improved apparatus according to claim 1 is provided for deploying a medical implant in a coronary sinus for repairing a defective mitral valve.

Described herein is a method of repairing a mitral valve comprises inserting a guidewire into a coronary sinus and advancing a distal end of a guide catheter along the guidewire to an ostium of the coronary sinus. A delivery catherter is advanced througf the guide catheter and into the coronary sinus. The delivery catheter includes an inner tubing and an outer sheath and a releasable attachment mechanism connecting the inner tubing and the outer sheath. The delivery catheter is configured to deliver a medical implant into the coronary sinus, wherein the medical implant includes a self-expanding proximal anchor, a self-expanding distal anchor and a bridge connecting the proximal and distal anchors. After advancing the delivery catheter, the releasable attachment mechanism is released and the outer sheath is retracted relative to the inner tubing to deploy the self-expanding distal anchor. The delivery catheter is withdrawn to remove slack in the bridge of the medical implant. The outer sheath is retracted further relative to the inner tubing to deploy the self-expanding proximal anchor. The distal anchor of the medical implant is preferably deployed in the anterior interventicular vein to ensure that the distil anchor is well secured.

In one variation, the outer sheath of the delivery catheter is retracted by proximally sliding a sliding button on a handle of the delivery catheter, wherein the sliding button and the outer sheath are fixedly attached. In another variation, the distal end of the guide catheter may be provided with an inflatable balloon. The inflatable balloon is preferably sized for placement in the ostium of the coronary sinus. The inflatable balloon may also be used as a sealing member, such that radiopaque fluid may be injected into the coronary sinus and contained within the coronary sinus before advancing the delivery catheter into the coronary sinus. In yet another aspect, the inflatable balloon on the distal end of the guide catheter may be inflated to increase a diameter of the coronary sinus before advancing the delivery catheter into the coronary sinus. To further enhance visualization, the inflatable balloon on the distal end of the guide catheter is preferably inflated with a radiopaque fluid

During delivery, the bridge of the medical implant is preferably positioned along an anterior wall of the coronary sinus before retracting the outer sheath. To assist in positioning the implant, the delivery catheter preferably includes at least one radiopaque marker band. The bridge of the medical implant is preferably made of a shape memory material with a resorbable material disposed along the bridge for maintaining the bridge in an extended condition during advancement of the delivery catheter into the coronary sinus. The length of the bridge contracts as the resorbable material is resorbed after deploying the proximal and distal anchors of the medical implant.

In one variation, the releasable attachment mechanism comprises a plurality of fingers along a distal end of the outer sheath and a flexible sleeve along a distal end of the inner tubing. The flexible sleeve is contractible over the plurality of fingers for holding the fingers in a friction-fit relationship.

According to the invention, the inner tubing of the delivery catheter further comprises an inflatable balloon along a distal end region. The inflatable balloon along the distal end region of the inner tubing may be inflated for engaging an inner wall of the outer sheath and deflated for disengaging an inner wall of the outer sheath, thereby providing the releasable attachment mechanism. In another feature, the inflatable balloon along the distal end region of the inner tubing may be configured to seat the distal anchor of the medical implant within the coronary sinus. In still another feature, the inflatable balloon along the distal end region of the inner tubing may be shaped to partially extend from a distal end of the outer sheath during advancement of the delivery catheter into the coronary sinus. A distal end portion of the inflatable balloon has a tapered shape for facilitating advancement of the delivery catheter. The distal end portion of the inflatable balloon may be coated with a lubricious coating.

Also described herein is a method of repairing a mitral valve which comprises providing a delivery catheter including an inner member and an outer sheath, wherein the inner member has an inflatable balloon disposed along a distal end region, the delivery catheter being configured to deliver a medical implant into a coronary sinus, the medical implant having proximal and distal anchors and a bridge connecting the proximal and distal anchors. A distal end of a guide catheter is advanced through a patient's vasculature and toward a coronary sinus. A distal end portion of the delivery catheter is advanced through the guide catheter and into the coronary sinus. The outer sheath is retracted relative to the inner member to expose the distal anchor. The inflatable balloon along the distal end region of the inner member is inflated to radially expand (i.e., seat) the distal anchor. The outer sheath is retracted relative to the inner member to expose the proximal anchor. If necessary, the inflatable balloon may also be used to help radially expand the proximal anchor.

Also described herein is a method of repairing a mitral valve comprises providing a delivery catheter having an inner member and an outer sheath, the delivery catheter being configured to deliver a medical implant into a blood vessel, the medical implant including a proximal anchor, a distal anchor and a bridge connecting the proximal and distal anchors. A distal end of the delivery catheter is advanced into an anterior interventricular vein. The outer sheath is then retracted relative to the inner member to deploy the distal anchor in the anterior interventricular vein. The outer sheath is retracted relative to the inner member to deploy the proximal anchor in a coronary sinus, preferably in the region close to the coronary ostium. After deployment, the medical implant (e.g., tension in the bridge) reshapes a mitral valve annulus for repairing the mitral valve. If desired, one or more stents may be deployed in the circumflex artery and/or left anterior descending artery before repairing the mitral valve to ensure patency of these arteries after the medical implant is deployed.

According to the present invention there is provided an apparatus for treating a mitral valve comprising a delivery catheter including an inner tubing and an outer sheath, the inner tubing having an inflatable balloon disposed along a distal end region. A handle is attached to a proximal end of the delivery catheter, the handle including a sliding button attached to the outer sheath. A self-expanding medical implant is located on the inner tubing in a contracted condition and is covered by the otter sheath. The sliding button is retractable for withdrawing the outer sheath and deploying the medical implant. The inflatable balloon preferably has a tapered distal end portion configured to extend from the outer sheath for facilitating advancement of the delivery catheter through a patient's vasculature and into a coronary sinus. The tapered distal end portion of the inflatable balloon may be coated with a lubricious coating. During delivery, at least a portion of the medical implant may be disposed over the inflatable balloon such that inflation of the inflatable balloon assists in the deployment of the medical implant.

Also described herein is a delivery system for deploying a medical implant in a coronary sinus comprising a guide catheter and a delivery catheter including an inner tubing and an outer sheath surrounding at least a portion of the inner tubing, the inner tubing having an attachment mechanism for engaging the outer sheath. A handle is attached to a proximal end of the delivery catheter. The medical implant is mounted on the inner tubing and is covered by the outer sheath during delivery to the coronary sinus. The handle is configured to withdraw the outer sheath relative to the inner tubing for deploying the medical implant. In one variation, the attachment mechanism comprises a flexible sleeve on the distal end of the inner tubing, wherein the flexible sleeve is sized to constrict around a distal end of the outer sheath. In another variation, the attachment mechanism comprises an inflatable balloon disposed along the distal end of the inner tubing for engaging an inner wall of the outer sheath.

### BRIEF DESCRIPTION OF THE DRAWINGS

Figure 1 is a three-dimensional view of the mitral valve and coronary sinus.

Figure 2 is a side view of an embodiment of a medical implant configured for delivery into a coronary sinus including a proximal anchor, a distal anchor and a bridge connecting the proximal and distal anchors.

Figure 3 is a schemantic view of the bridge of Figure 2.

Figure 4a is a side view of a guide catheter and a dilator which form a portion of a delivery system for delivering a medical implant.

Figure 4b is a side view illustrating a guide catheter having an inflatable member disposed along a distal end region.

Figure 4c is a side view of the dilator shown in Figure 4a.

Figure 5 is a side view of a delivery catheter which is configured for advancement through the guide catheter for delivering a medical implant to the coronary sinus.

Figure 6 is a side view of the inner shaft assembly which forms a part of the delivery catheter of Figure 5.

Figure 6a is a side view of an inner shaft assembly according to the present invention having an inflatable balloon disposed along the distal end region.

Figure 6b is a cross-sectional view of the inner shaft assembly of Figure 6a illustrating guidewire and inflation lumens in the inner tubing.

Figure 6c is a partial cut-away side view illustrating the inner shaft assembly of Figure 6a in combination with the outer sheath.

Figure 6d is a side view of the inner shaft assembly of Figure 6a wherein the outer sheath is retracted and wherein the balloon has been inflated to expand the distal anchor of the medical implant.

Figure 7 is a side view of the outer shaft assembly which forms a portion of the delivery catheter of Figure 5.

Figure 8 is a side cross-sectional view of the handle of the delivery catheter of Figure 5.

Figure 9 is a perspective view of a sliding button configured for use with the handle of the present invention.

Figure 10 is an enlarged view illustrating a distal end portion of the delivery catheter of Figure 5.

Figure 11 illustrates a guidewire inserted into the coronary sinus for facilitating advancement of the delivery system.

Figure 12 illustrates a guide catheter and a dilator advanced over the guidewire to the coronary sinus.

Figure 13 illustrates the delivery catheter of Figure 5 advanced over the guidewire into the coronary sinus.

Figure 14 illustrates the delivery catheter of Figure 5 wherein the outer sheath has been partially retracted to release the distal anchor of the medical implant.

Figure 14a illustrates the delivery catheter having the alternative inner shaft assembly of Figure 6a wherein the outer sheath has been partially retracted to release the distal anchor and the balloon has been inflated for assisting in the deployment of the distal anchor.

Figure 15 illustrates the medical implant after the outer sheath of the delivery catheter has been fully retracted to release both the proximal and distal anchors.

Figure 16 illustrates a medical implant after delivery in the coronary sinus wherein the mitral valve annulus has been reshaped by the implant for reducing regurgitation in the mitral valve.

Figure 17 is a perspective view illustrating one preferred placement of a medical implant for treating a mitral valve.

Figures 18a and 18b are perspective views illustrating the placement of a medical implant for treating a tricuspid valve.

Figure 19 schematically illustrates a distal anchor expanded in the proximal region of the anterior interventricular vein for treating a mitral valve as shown in Figure 17.

Figure 20 schematically illustrates a distal anchor expanded distal within the proximal region of the anterior interventricular vein according to an alternative delivery method.

Figure 21 illustrates the delivery catheter of Figure 5 used in combination with the alternative guide catheter of Figure 4b wherein the balloon on the guide catheter is inflated within the coronary ostium.

### DETAILED DESCRIPTION

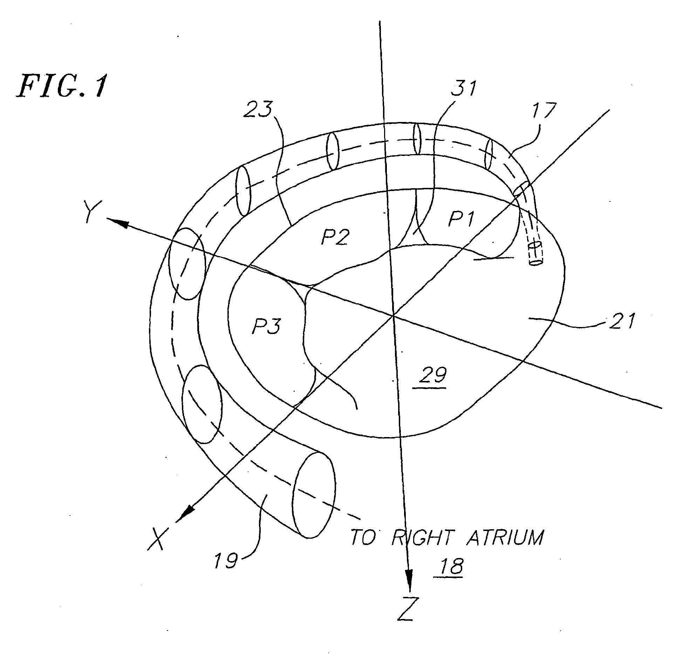

With reference now to Figure 1, a three-dimensional view of a mitral valve 21 and a coronary sinus 17 is provided for background purposes. From this view, it can be seen that the coronary sinus extends around a posterior region of the mitral valve 21. The coronary sinus is a relatively large vessel that receives venous drainage from the heart muscle. Blood flows through the coronary sinus and empties into the right atrium 18 through a coronary ostium 19. A mitral annulus 23 is a portion of tissue surrounding a mitral valve orifice to which the valve leaflets attach. The mitral valve 21 generally includes an anterior leaflet 29 and a posterior leaflet 31. The posterior leaflet is formed with three scallops P1, P2 and P3. As used herein, the term coronary sinus 17 is used as a generic term that describes the portion of the vena return system that is primarily situated adjacent to the mitral valve 21 and extends, at least in part, along the atrioventricular groove. Accordingly, the term "coronary sinus" may be construed to include the great cardiac vein and all other related portions of the vena return system.

It has been found that dilation of the mitral valve annulus 23 is the primary cause of regurgitation (i.e., reversal of flow) through the mitral valve 21. More particularly, when a posterior aspect of the mitral annulus 23 dilates, one or more of the posterior leaflet scallops P1, P2 or P3 moves away from the anterior leaflet 29. As a result, the anterior and posterior leaflets fail to close completely and blood is capable of flowing backward through the resulting gap. To reduce or eliminate mitral regurgitation, it is desirable to move the posterior aspect of the mitral annulus 23 in an anterior direction, thereby closing the gap caused by the leaflet displacement.

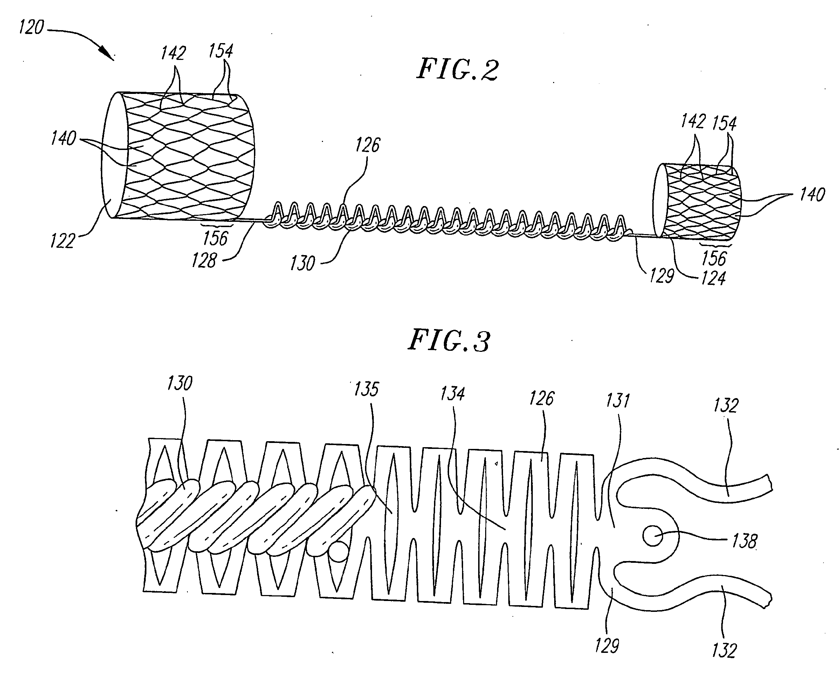

With reference now to Figures 2 and 3, one preferred embodiment of a medical implant 120 is provided for repairing a defective mitral valve. The implant is configured for deployment in the coronary sinus and is shaped to press against the posterior aspect of the mitral annulus. Embodiments of the delivery system, as will be described in more detail below, are particularly well-suited for delivering this type of implant into the coronary sinus. As illustrated, the implant 120 includes a proximal anchor 122 and a distal anchor 124 connected by a bridge 126. As used herein, "distal" means the direction of a device as it is being inserted into a patient's body or a point of reference closer to the leading end of the device as it is inserted into a patient's body. Similarly, as used herein "proximal" means the direction of a device as it is being removed from a patient's body or a point of reference closer to a trailing end of the device as it is inserted into a patient's body. The bridge 126 is configured to foreshorten for drawing the proximal and distal anchors together after the implant has been deployed. A resorbable material is disposed within openings 135 in the bridge. The resorbable material holds the bridge in an elongated state during delivery and deployment. However, over time, the material is resorbed such that the bridge is allowed to shorten.

Resorbable materials are those that, when implanted into a human body, are resorbed by the body by means of enzymatic degradation and also by active absorption by blood cells and tissue cells of the human body. Examples of such resorbable materials are PDS (Polydioxanon), Pronova (Poly-hexafluoropropylen-VDF), Maxon (Polyglyconat), Dexon (polyglycolic acid) and Vicryl (Polyglactin). As explained in more detail below, a resorbable material may be used in combination with a shape memory material, such as Nitinol, Eigiloy or spring steel to allow the superelastic material to return to a predetermined shape over a period of time.

As shown in Figure 2, the proximal and distal anchors 122, 124 are both generally cylindrical and are made from tubes of shape memory material, such as, for example, Nitinol. However, the anchors 122, 124 may also be made from any other suitable material, such as stainless steel. In the illustrated example, both anchors 122, 124 have a mesh configuration comprising loops 154 of zig-zag shaped shape memory material having alternating peaks 142. The loops 154 are connected at each peak 142 to form rings 156 of four-sided openings 140. Although one particular type of anchor mechanism is shown for purposes of illustration, it will be appreciated that a wide variety of anchoring mechanisms may be used.

The proximal and distal anchors 122, 124 each have a compressed state and an expanded state. In the compressed state, the anchors 122, 124 have a diameter that is less than the diameter of the coronary sinus 17. In the compressed state, the anchors 122, 124 preferably have a substantially uniform diameter of between about 1.5 mm and 4 mm. In the expanded state, the anchors 122, 124 have a diameter that is preferably about equal to or greater than a diameter of the section of a non-expanded coronary sinus 17 to which each anchor will be aligned. Since the coronary sinus 17 has a greater diameter at its proximal end than at its distal end, in the expanded state, the diameter of the proximal anchor 122 is preferably between about 10 mem and 18 mm and the diameter of the distal anchor 124 is preferably between about 3 mm and 8 mm.

The bridge 126 is connected between the proximal anchor 122 and distal anchor 124 by links 128, 129. More specifically, as shown in Figure 2, a proximal link 128 connects the proximal stent section 122 to a proximal end of the bridge 126 and a distal link 129 connects the distal stent section 124 to a distal end of the bridge 126. The links 128 and 129 have a base 131 and arms 132 that extend from the base and which are connected to two peaks 142 on each anchor 122, 124. Further, the links 128, 129 may be provided with a hole 138, as shown in Figure 3, which serves as a means through which to pass the end of the resorbable thread and secure it to the bridge 126.

The bridge 126 is preferably made from a shape memory material and is sufficiently flexible to allow the implant 120 to conform to the shape of the coronary sinus 17. The bridge 126 comprises X-shaped elements 134 wherein each X-shaped element is connected to an adjacent X-shaped element at the extremities of the "X," allowing a space 135 to be created between adjacent X-shaped elements, as shown in Figure 3. The X-shaped elements 134 further have rounded edges that minimize the chance that a sharp edge of the bridge 126 will puncture or cut a part of the coronary sinus 17 during delivery. The bridge 126 has two states: an elongated state in which the bridge has a first length, and a shortened state in which the bridge has a second length, the second length being shorter than the first length. As discussed above, resorbable thread 130 is woven into the spaces 135 (shown schematically in Figures 2 and 3) between adjacent X-shaped elements 134 to hold the bridge 126 in its elongated state. The thread 130 acts as a temporary spacer. When the resorbable thread 130 is dissolved over time by means of resorption, the bridge assumes its shortened state. The above-described implant 120 is one example of a mitral valve repair device that may be used in accordance with the delivery system of the present invention. However, it will be appreciated that the delivery system may be used with a variety of mitral valve repair devices. For example, the delivery system may be configured for use with the mitral valve repair devices described in U.S. Patent Application No. 11/014,273, filed December 15, 2004.

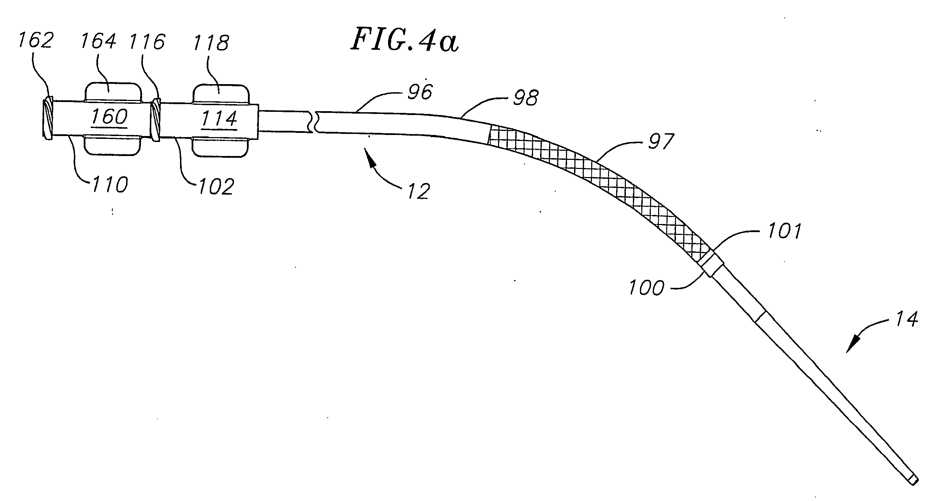

Figures 4a through 5 illustrate a delivery system for delivering the medical implant into a coronary sinus includes, generally, a guide catheter 12, a dilator 14 and a delivery catheter 16. As will be described in more detail below, the dilator 14 is inserted through the guide catheter 12 (as shown in Figure 4a) and both components are advanced over a guidewire toward the coronary sinus. After a path to the coronary sinus 17 has been established, the dilator 14 is withdrawn and removed from the venous system. The delivery catheter 16 on which the medical implant 120 is disposed is then advanced through the guide catheter 12 for delivering the medical implant into the coronary sinus.

With particular reference to Figure 4a, the guide catheter 12 preferably includes a braid-reinforced composite tube having a fitting 102 at a proximal end. In one embodiment, the composite tube generally includes a polytetrafluoroethylene (PTFE) liner, a braid that extends the length of the shaft to a guide catheter marker band 101, and a first coated region 96. The braid of the guide catheter 12 provides the guide catheter with the requisite stiffness to allow it to be pushed through a patient's vasculature and also allows the shaft to maintain a substantially cylindrical shape. The braid is preferably formed of a metallic material, such as, for example, stainless steel. Along the coated region, the braid is encapsulated by an external layer of tubing material that may vary along the length of the shaft. For example, in one preferred construction, a proximal section of the shaft is made from PEBAX 7233, a mid-section is made from PEBAX 5533 and a distal section is made from PEBAX 3533. The first coated region 96 provides additional rigidity to the guide catheter 12 to allow it to be more easily pushed through a patient's vasculature.

Distal to the first coated region 96, the guide catheter 12 includes a second coated region 97 that is preferably more flexible than the first coated region. The second coated region 97 extends to the guide catheter marker band 101 located near a distal end of the guide catheter 12. The guide catheter marker band 101 is visible under fiuoroscopy, thereby allowing the relative position of the guide catheter to be tracked as the guide catheter is advanced through the venous system. Distal to the marker band 101 is an unbraided region 100 which provides a flexible atraumatic distal tip. The unbraided region 100 allows the guide catheter 12 to be advanced distally into the coronary sinus 17 as is described in more detail below.

The fitting 102 located along the proximal end of the guide catheter 12 includes a rigid plastic body 114, which may be attached by an adhesive, and a threaded flange 116 at a proximal end of the body. The inside of the body 114 may be tapered distally toward a central axis of the body. Additionally, wings 118 may extend perpendicularly from the body 114 that act as finger grips

The guide catheter 12 is preferably formed with a curved or bent distal region 98. In one example, the curved or bent distal region 98 may be advantageously used to facilitate access to the coronary sinus 17, such as, for example, from the femoral vein. The shape of the bent distal region 98 may also assist in orienting the medial implant during delivery and deployment. More specifically, the guide catheter is curved to conform to the anatomy in the region of the coronary sinus. Thus, when the delivery catheter is advanced through the guide catheter and into the coronary sinus 17 with the implant 120 mounted thereon, as is described in more detail below, the position of the guide catheter ensures that the implant is properly oriented.

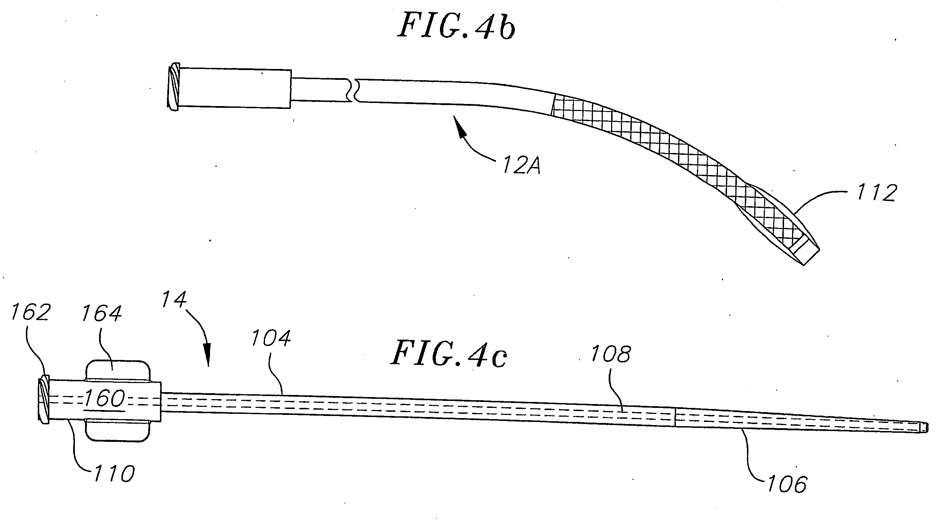

With reference now to Figure 4b, an alternative example of a guide catheter 12A is illustrated wherein an expandable member is disposed along a distal end region. In the illustrated embodiment, the expandable member takes the form of an inflatable balloon 112 having a compressed stated and an expanded state. In one application, the balloon 112 may be used to assist in anchoring the guide catheter 12 relative to the coronary sinus 17, thereby reducing or eliminating axial movement of the guide catheter when various components of the delivery catheter are inserted through the guide catheter. If desired, the balloon may be inflated using a radiopaque fluid to improve visualization of the distal end region of the guide catheter 12A. The balloon 112 may also be used to dilate the coronary ostium and/or the coronary sinus in a controlled manner to reduce the risk of tearing the coronary sinus and to facilitate advancement of the implant. In yet another advantageous feature, the balloon may be used as a sealing member to block the coronary ostium before injecting radiopaque fluid into the coronary sinus. This feature may be used to improve visualization before, during or after the delivery of the medical implant.

With reference now to Figure 4c, the dilator 14 is a relatively rigid composite tubular member having a body 104 and a tapered distal region 106. The dilator 14 is configured for insertion into a central lumen extending through the guide catheter 12, 12A. The dilator 14 has a guidewire lumen 108 for allowing the dilator to be advanced over the guidewire 82. The body 104 of the dilator 14 is preferably made from HDPE and the distal region 106 is made from low density polyethylene (LDPE) and barium sulfate (BaSO4), which is added to provide radiopacity. The tapered distal region 106 is more flexible than the body 104 for allowing the distal region to be navigated through a patient's vasculature without damaging the vessel walls.

The dilator 14 preferably includes a fitting 110 at its proximal end. The fitting 110 includes a rigid plastic body 160, attached to the body 104 of the dilator 14 by an adhesive, and a threaded flange 162 at a proximal end of the body. The inside of the body 160 is tapered inwardly to guide a tube inserted into the fitting 110 to a central axis of the fitting.

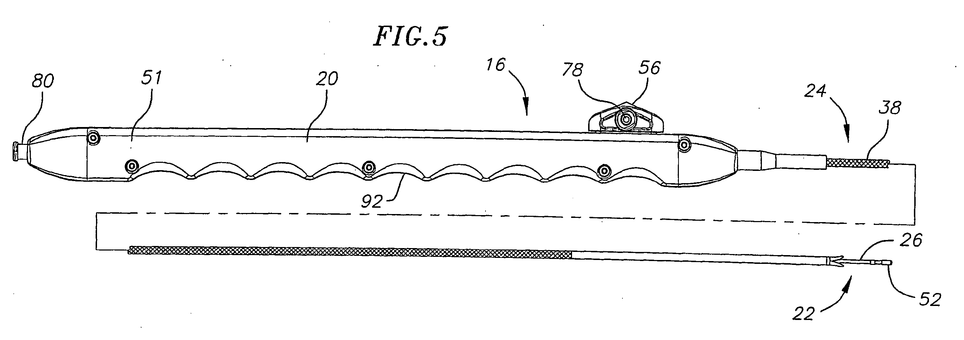

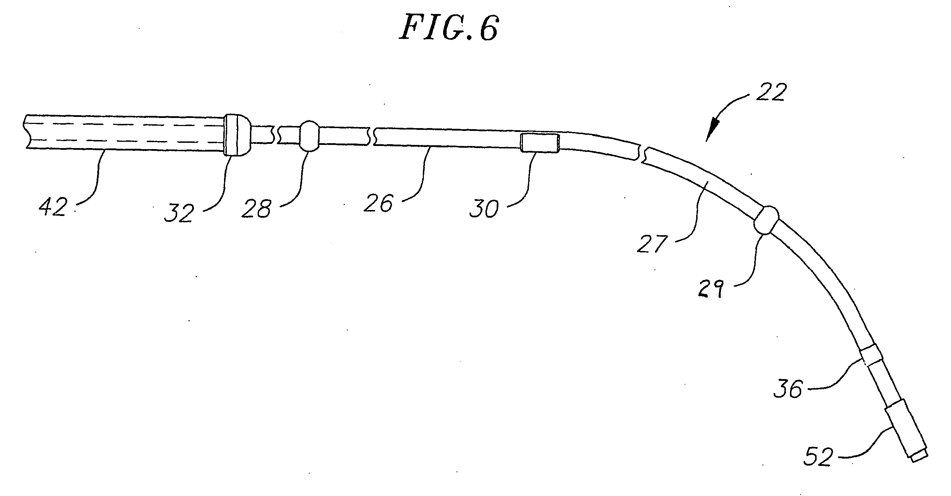

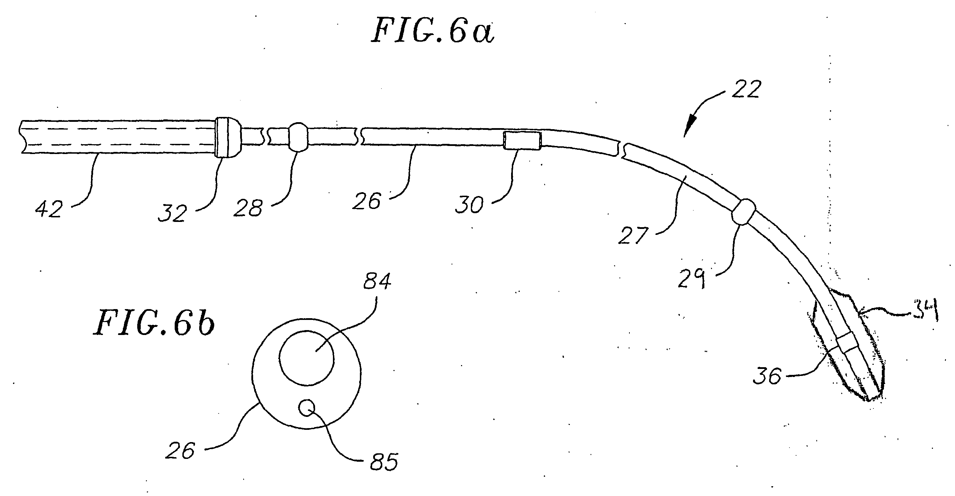

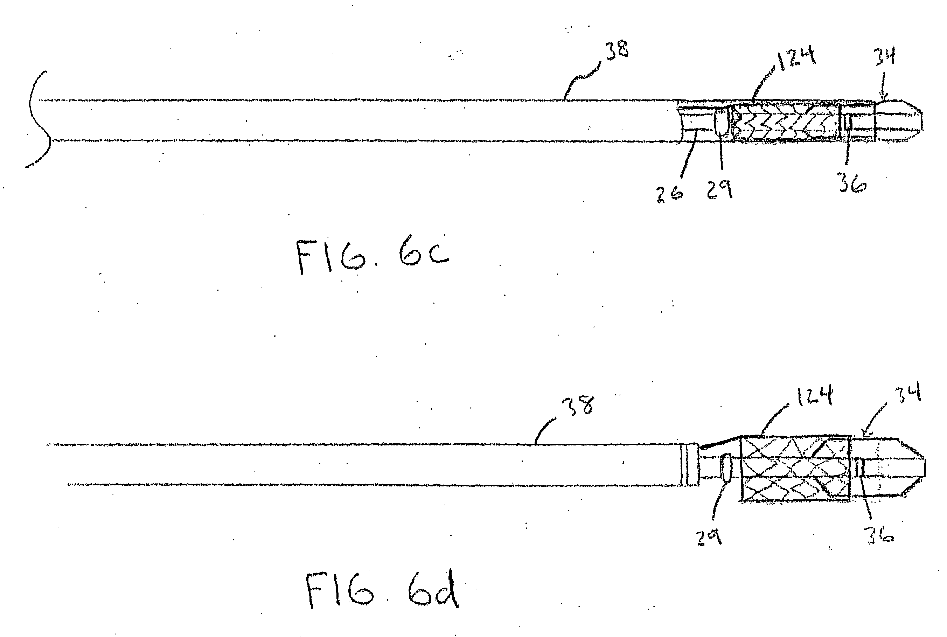

With reference now to Figures 5 and 6, the delivery catheter 16 generally includes a handle portion 20, an inner shaft assembly 22 and an outer shaft assembly 24. The delivery catheter 16 provides a means for advancing the medical implant through the guide catheter and into the coronary sinus. The handle portion 20 of the delivery catheter provides an actuation mechanism for effecting relative movement between the inner and outer shaft assemblies for releasing the medical implant after reaching the treatment site. With particular reference to Figure 6, the inner shaft assembly 22 of the delivery catheter generally includes an inner tubing 26 having a small diameter and a pusher tube 42 having a larger diameter. The inner shaft assembly 22 preferably extends distally about 42 inches and has a lumen 27 adapted to receive a guidewire. As will be described in more detail below, the medical implant is disposed on the inner tubing 26 during delivery. The inner tubing 26 is preferably formed with a diameter selected to allow the tubing to be flexible and have a minimal profile, yet to still maintain structural integrity as it is navigated through the vasculature of a patient. In one example, the inner tubing 26 is made from thin wall polyetheretherketone (PEEK). However, the inner tubing 26 may also be made from, for example, a tri-layer plastic tubing having a high density polyethylene (HDPE) liner or any other flexible biocompatible material able to be formed into a thin tube. Preferably, the inner diameter of the inner tubing 26 is about 0.040 inch.

With continued reference to Figure 6, the inner tubing 26 is preferably provided with a series of marker bands. The inner tubing 26 includes two C-shaped bands 28, 29 a mid-bridge locater band 30, and proximal and distal O-shape bands 32, 36. The marker bands 28, 29, 30, 32, 36 are visible under fluoroscopy. The medical implant is preferably fixed with respect to the marker bands during delivery. As a result, the marker bands allow the operator to visualize the location of the implant and thereby serve to assist in the placement of the medical implant within the coronary sinus. The proximal O-shaped band 32 is located at a distal end of the pusher tube 42. The distal O-shaped band 36 is located near or adjacent a distal end of the inner tubing 26. When used with the medical implant 120 described above with respect to Figures 2 and 3, the proximal anchor 122 of the medical implant is preferably located between the proximal O-shaped band 32 and the proximal C-sbaped band 28. The distal anchor 124 of the medical implant is preferably located between the distal C-shaped band 29 and the distal O-shaped band 36. The bridge 126 of the medical implant passes through the gaps in the C-shaped bands 28, 29 and through the gap in the mid-bridge locator band 30. The gaps in the C-shaped bands and mid-bridge locator may be rotationally oriented on the inner tubing to hold the bridge in a particular position relative to the inner tubing. Accordingly, the orientation of the marker bands 28, 29, 30, 32, 36 may be used to ensure that the bridge and the anchors are in the proper rotational and longitudinal positions before deployment. This is particularly advantageous because it may be desirable to deliver the medical implant in a position wherein the proximal and distal anchors are not rotationally aligned in order to better conform to the complex contour of the coronary sinus. In one example, the proximal and distal O-shaped markers 32, 36 may be 90/10 platinum/iridium marker bands. However, the marker bands 28, 30, 32, 36 may also be made of, for example, stainless steel, or any other biocompatible material that is visible under fluoroscopy. A luer fitting (not shown) may be located at a proximal end of the inner tubing 26 to allow flushing of the guide wire lumen 27 prior to a procedure using the delivery system.

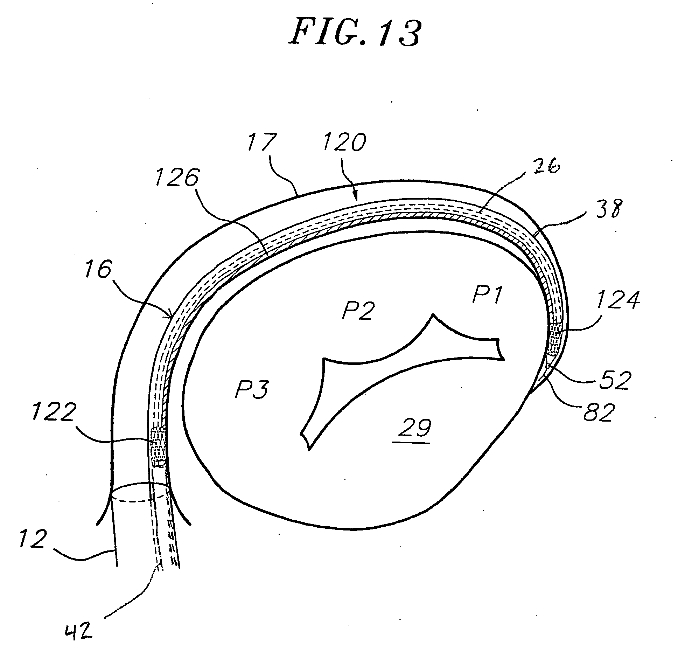

The pusher tube 42 is fixed to and extends from a distal end of the handle position 20 to the proximal O-shaped band 32. The pusher tube 42 provides structural support to the inner and outer shaft assemblies 22, 24 and also provides an interface with an outer sheath 38 of the outer shaft assembly. The proximal marker 38 is preferably located on the distal end of the pusher tube and abuts the proximal end of the medical implant. Accordingly, the pusher tube 42 may also provide a pusher mechanism for resisting undesirable longitudinal movement of the medical implant during retraction of the outer shaft assembly. The pusher tube 42 may be made from HDPE with a thickness of about 0.0165 inch and a diameter of about 0.089 inch. The natural low coefficient of friction of HDPE tubing minimizes friction between the outer tube 42 and the retractable outer sheath 38 during deployment of the implant 120 (see Figure 13) which is secured between the outer sheath and the inner tubing 26 of the inner shaft assembly 22.

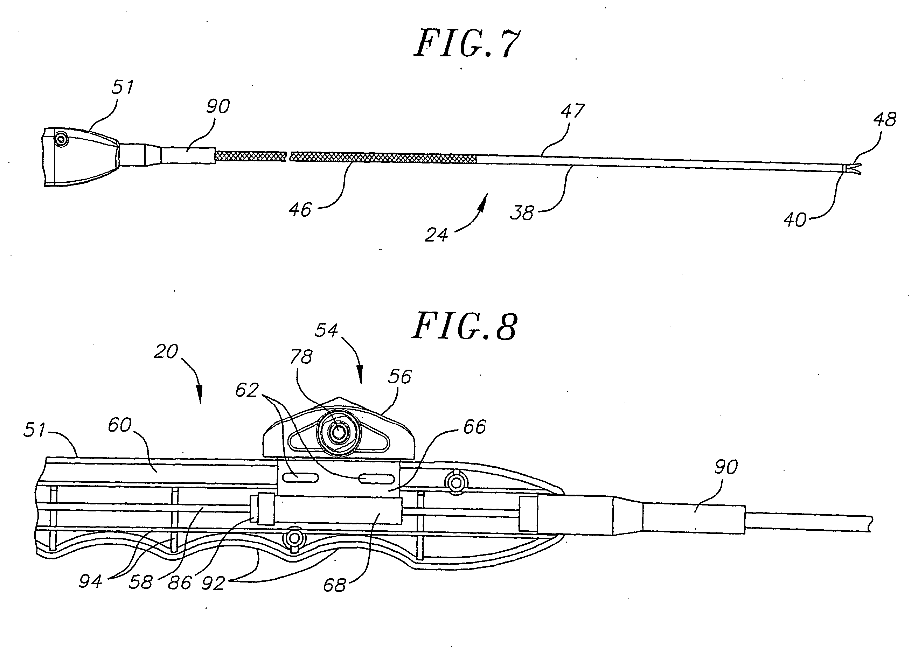

With reference to Figure 7, the outer shaft assembly 24 of the delivery catheter preferably comprises a composite outer sheath 38 including a braided region 46, an unbraided region 47, and a distal fastening region 48. The outer sheath 38 is sized to surround and contain the medical implant during advancement to the coronary sinus, thereby holding the proximal and distal anchors of the medical implant in the contracted position and protecting the vessel walls from damage. The outer sheath 38 may be made from a material that has a relatively low coefficient of friction and is compatible with e-beam radiation sterilization (i.e. the material properties are not adversely affected by exposure to radiation sterilization).

The outer sheath 38 preferably includes an inner liner made from HDPE and an outer layer made from 50% HDPE and 50% LDPE (50/50 HDPE/LDPE) that encapsulate a braided region 47 made from stainless steel. The 50/50 HDPE/LDPE layer extends up to the marker band 40 while the HDPE liner extends the entire length of the outer sheath 38. The unbraided section 47 may be transparent to allow visualization of, for example, the implant 120 within the sheath, as will be described below. The braided region 46 is located on a proximal portion of the outer sheath 38 and the braids may be made from, for example, stainless steel. The braided region 46 provides the outer shaft assembly 24 with the requisite stiffness to allow the delivery device to be pushed through a patient's vasculature.

A distal fastening region 48 of the outer sheath 38 includes an outer sheath marker band 40, the marker band being visible under fluoroscopy. The location of the marker band 40 on the distal region 48 of the outer sheath 38 allows the relative displacement between the outer sheath and the inner shaft assembly 22 to be tracked. This ability to visualize the outer sheath 38 permits a more controlled retraction of the sheath and thus, a more controlled deployment of the implant 120.

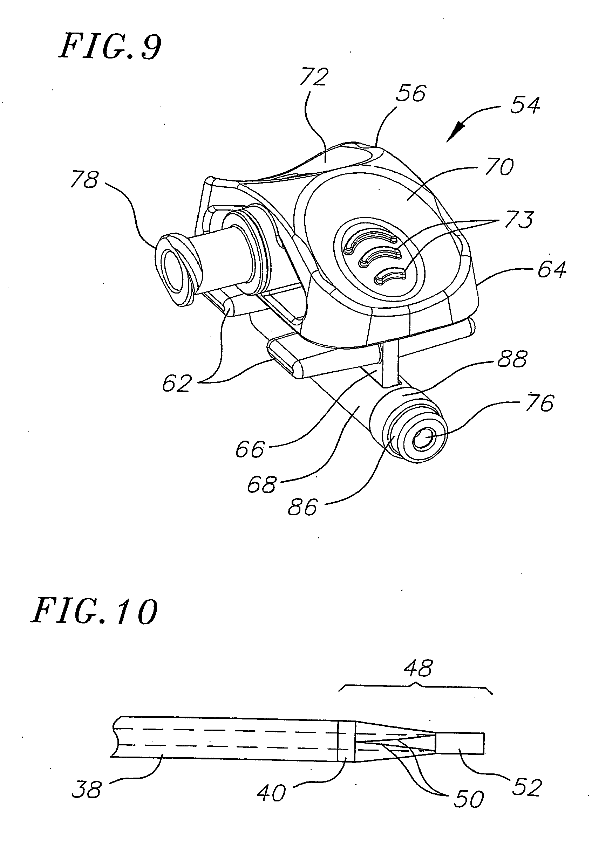

During delivery, the distal fastening region 48 of the outer sheath 38 is preferably tapered for providing a smooth transition from the inner tubing 26 to the outer sheath 38. As best shown in Figure 10, the distal region 48 of the outer sheath 38 preferably includes a plurality of flexible fingers 50 separated by slits. The slits on the distal region 48 allow the outer sheath 38 to be tapered and to conform to the smaller diameter of the inner tubing 26. In another advantageous feature, the tapered distal region 48 is less bulky and more flexible, making the overall catheter tip more trackable. Additionally, the resulting spaces in the distal region 48 allow the device to be flushed as is described in more detail below.

With reference to Figures 6 through 10, a flexible sleeve 52 may be provided along the distal end portion of the inner shaft assembly 22 for gripping and holding the fingers 50 of the outer sheath. The sleeve 52, which may be formed of silicone, is preferably attached by an interference fit and an adhesive to the inner tubing 26 such that a distal end of the sleeve is substantially flush with the distal end of the tubing. A proximal portion of the sleeve 52 is configured to be stretched and positioned over a segment of the distal region 48 of the outer sheath 38 to releasably secure the outer sheath 38 to the inner tubing 26. More particularly, as best illustrated in Figure 10, the sleeve 52 is configured to be positioned over at least a portion of the fingers 50 which are disposed along the distal region 48 of the outer sheath 38. The sleeve provides a releasable attachment mechanism for securing the distal region 48 of the outer sheath 38 to the inner tubing 26. The releasable attachment mechanism prevents the outer sheath from inadvertently retracting during advancement of the delivery catheter 16 to the treatment site. The releasable attachment mechanism also provides a smooth transition region between the outer sheath and inner tubing. If desired, an adhesive may be used to enhance the attachment between the distal region 48 of the outer sheath 38 and the inner tubing 26. Alternatively, or in addition, the releasable attachment mechanism may take the form of a break-away mechanism or any other suitable device. With reference again to Figure 10, when the outer sheath 38 is retracted with sufficient force relative to the inner tubing 26, the fingers 50 are pulled free from the sleeve 52. After the fingers are released, the sleeve preferably collapses over the outer diameter of the inner tubing 26, thereby permitting the sleeve to retain a minimal cross-sectional profile. The minimal profile reduces the potential for the sleeve 52 to snag or otherwise interfere with the implant 120 when the inner shaft assembly 22 is removed from a patient after delivering the implant.

According to the present invention, and with reference to Figures 6a and 6b, the inner shaft assembly 22 is provided with an expendable member 34 along a distal end region. The expandable member preferably takes the form of an inflatable balloon which may be configured for a variety of purposes. In order to accommodate the inflatable balloon, the inner tubing 26 preferably has a dual lumen structure as shown in Figure 6b. A guidewire lumen 84 is adapted to receive a guidewire 82 and an inflation lumen 85 is provided as a conduit for inflation fluid to reach the expendable member. In one application, the expandable member 34 is preferably shaped with a tapered distal end to provide a smooth atraumatic tip which facilitates advancement of the delivery catheter to the treatment site. In another application, the expandable member 34 may be used as a releasable attachment mechanism for holding the outer sheath. More particularly, the expandable member may be sized to frictionally engage the inner wall of the outer sheath for preventing the outer sheath from inadvertently retracting relative to the inner tubing during delivery.. Accordingly, this embodiment provides an alternative releasable attachment mechanism which may be used instead of the fingers and flexible sleeve described above. In yet another application, the expandable member 34 may be situated such that at least a portion of the distal anchor of the medical implant is disposed over the expandable member during delivery. For example, that expandable member is preferably located such that approximately half of the distal anchor 124 of the medical implant 120 is located over the expandable member during delivery. In alternative embodiments, more or less of the anchor may be placed over the expandable member.

According to the present invention, and with reference to the partial cut-away view of Figures 6c, it can be seen that the expandable member 34 is disposed along the distal end region of the inner tubing 26. The expandable member 34 is an inflatable balloon comprising three portions. A proximal portion of the balloon is surrounded by the contracted distal anchor 124. A central portion of the balloon is located distal to the distal anchor for engaging the inner wall of the outer sheath 38, thereby preventing the outer sheath from sliding relative to the inner tubing 26. A distal portion of the balloon extends from the distal tip of the outer sheath 38 and is shaped to provide a smooth transition region between the outer sheath and the inner tubing. Preferably, the balloon has a length of approximately 2 cm with a wall thickness of about 0.001 inches. The exposed distal portion of the balloon may include a lubricious coating, such as, for example, a hydrophilic or silicone coating, to reduce friction and thereby facilitate advancement of the delivery catheter through the patient's vasculature. The balloon is preferably formed of a semi-compliant material, such as, for example, PEBAX or urethane. Preferably the balloon is configured such that the exposed distal portion of the balloon has a diameter during delivery that is substantially equal to the diameter of the outer sheath to further facilitate advancement.

After reaching the treatment site, the expandable member is deflated to disengage the inner wall of the outer sheath 38, thereby allowing the outer sheath to be retracted relative to the inner tubing. With reference now to Figure 6d, after the outer sheath has been sufficiently retracted to expose the distal anchor 124, the balloon may be re-inflated to help radially expand the distal anchor 124, thereby ensuring that the distal anchor is properly seated in the coronary, sinus.

With reference now to the cross-sectional view of Figure 8, the handle portion 20 of the delivery device includes a handle body 51 and a sliding button assembly 54. The handle body 51 has two symmetrical halves having a length sufficient to allow for controlled retraction of the outer sheath 38 for implants having a wide array of lengths. The handle body 51 also has a series or ergonomic arches 92 formed along one edge hallowing for a more secure grip. Preferably, the length of the handle portion 20 is about 30 cm. The interior of the bundle body 51 includes a groove 60 adapted to receive guiding wings 62 attached to a sliding button 56 as described in more detail below. The interior of the handle body 51 may also contain ribs 94 running perpendicularly along its length and width to make the handle body more resistant to bending and permanent deformation. The sliding button assembly 54 includes the sliding button 56 and a rail 58. Additionally, the sliding button 56 has a transition region 66 and a sliding adaptor 68.

With reference to Figure 9, an enlarged perspective view of the sliding button assembly 54 is shown. The sliding button 56 is formed with a generally triangular shape and includes an advancing surface 70 and a retracting surface 72 for moving the sliding button 56 along the handle portion. The advancing and retracting surfaces 70, 72 are ergonomically shaped and have centrally-disposed ridges 73 to allow controlled movement of the sliding button 56. The transition region 66 is rectangular and extends perpendicularly from a flat surface of the sliding button 56 opposite the advancing and retracting surfaces 70, 72. Extending perpendicularly from the transition region 66 are guiding wings 62 which are adapted to fit into the groove 60 on the handle portion 20.

Attached to the transition region 66 of the sliding button 56 is the sliding adaptor 68 which is adapted to be slidably connected along the rail 58. In one preferred embodiment, the sliding adaptor 68 is a cylindrical body having a central lumen 76 through which the rail 58 passes. A proximal end of the sliding adaptor 68 has an end cap 86 (see Figure 8) and an O-ring housing portion 88 containing an O-ring proximally adjacent to the end cap 86. The proximal end of the outer shaft assembly 24 is inserted and attached to the lumen 76, thereby allowing movement of the outer sheath assembly to correspond to movement of the sliding button 56.

The sliding button 56 also includes a flushport 78 which provides a passage from the sliding button to the lumen between the inner shaft assembly 22 and the outer sheath 38. The flushport 78 allows flushing of the lumen between the inner shaft assembly 22 and the outer sheath 38 without interfering with the relative displacement of the inner shaft assembly with respect to the outer sheath. The flushport 78 further incorporates a check valve (not shown) that allows inward flow of, for example, a saline solution, but prevents outward flow of, for example, blood during the operation of the delivery system.

At its proximal end, the rail 58 has a guidewire lumen flushport 80 (see Figure 5) which allows a flushing solution such as saline solution to flush a guidewire lumen (not shown) that runs the length of the handle portion 20 and the delivery catheter 16. A proximal end of the handle body 51 is adapted to hold the guidewire lumen flushport 80, which may be attached to the body by an adhesive. A strain relief sleeve 90 (see Figure 8) is attached to a distal end of the handle portion 20 and extends distally over part of the outer sheath assembly 24 to provide additional support and resistance to bending at the junction between the handle portion 20 and the outer sheath 38. Additionally, the strain relief sleeve 90 provides a smooth tapered transition between the handle body 51 and the outer sheath assembly 24. A distal end of the handle portion 20 is adapted to hold the strain relief assembly which may be attached to the handle portion 20 by an adhesive.

As best illustrated in Figure 13, the medical implant 120 is loaded onto the delivery catheter 16 before use. More specifically, the proximal and distal anchors 122, 124 are collapsed into their compressed state onto the inner tubing 26 such that the inner diameter of the anchors is approximately equal to the outer diameter of the inner tubing. First, the proximal anchor 122 is collapsed and the outer sheath 38 is distally advanced over the proximal anchor. Then, the bridge 126 is positioned on the inner shaft assembly 22 and the outer sheath 38 is advanced over the bridge. Using the radiopaque markers on the inner tubing, the orientation of the bridge 126 with respect to the delivery device may be noted using fluoroscopy such that an operator of the delivery device can place the bridge in a desired location within the coronary sinus as is described in more detail below. Finally, the distal anchor 124 is collapsed and the outer sheath 38 is advanced to its final position.

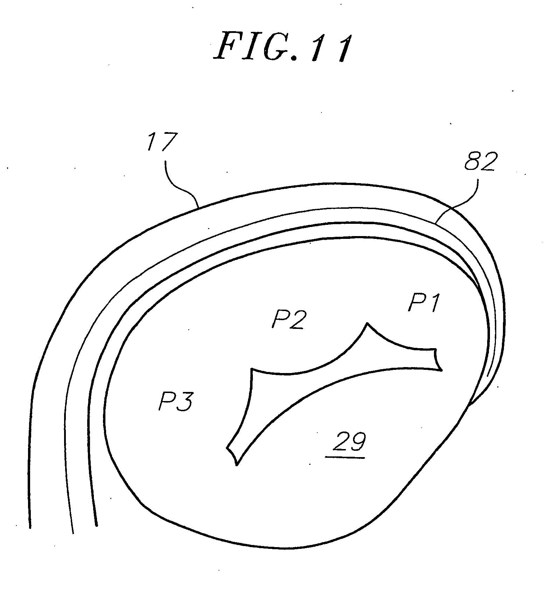

With reference now to Figures 11 through 16, preferred methods of delivering and deploying a medical implant in a coronary sinus will be described in more detail. First, the patient is prepared and an introducer sheath (not shown) may be inserted into a left or right internal jugular vein or the femoral vein which provides access to the coronary sinus as is generally known in the art. After the introducer sheath is secured, the guidewire 82 is inserted through the introducer sheath and into the coronary sinus (i.e., into the great cardiac vein), as shown in Figure 11. After a distal end of the guidewire has been placed distally in the coronary sinus 17, the guide catheter 12 and dilator 14 may then be prepared to be inserted. First, a syringe filled with flushing fluid, such as heparinized saline solution, may be used to flush the dilator 14 and the guide catheter 12 to remove any residual air and improve lubricity. A hemostatic valve, such as a Y-connector, is attached to a proximal end of the guide catheter 12. The hemostatic valve minimizes blood loss through an interface between the guide catheter 12 and other devices loaded through the guide catheter.

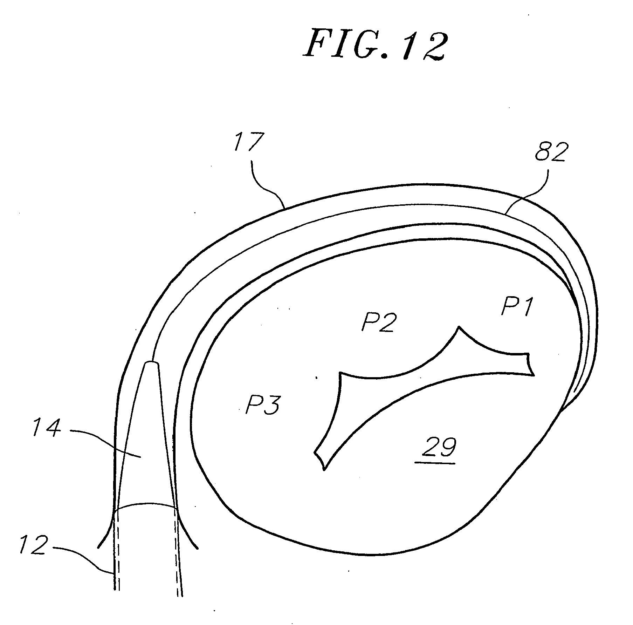

With reference again to Figure 4a, the dilator 14 is inserted through the central lumen of the guide catheter 12 such that the fitting 110 of the dilator is proximally adjacent the fitting 102 of the guide catheter. The dilator 14 serves to provide a smooth transition between the relatively small diameter guidewire 82 and the relatively large diameter guide catheter 12, thereby reducing the likelihood that a leading edge of the guide catheter will damage the vasculature of a patient as the guide catheter is inserted. As shown in Figure 12, the guide catheter 12 and dilator 14 are placed onto the guidewire 82 and advanced over the guidewire under fluoroscopy until a distal end of the guide catheter 12 is position at a desired location in the coronary sinus 17. The dilator 14 is then withdrawn proximally from the guide catheter.

The delivery catheter 16 may be flushed with a flushing fluid before it is inserted into a patient. Flushing fluid is inserted through the flushport 78 (see Figure 8) on the sliding button 56. The flushport 78 incorporated onto the sliding button 56 allows the flushport to move with the outer shaft assembly 24 and allows unrestricted relative displacement between the inner and outer shaft assembly 22, 24. The end cap 86 and the O-ring (not shown) within the sliding adaptor 68 provide a hemostasis function preventing blood from entering the handle portion while permitting the flushing fluid to be inserted between the inner shaft assembly 22 and the outer shaft assembly 24. As shown in Figure 10, the slits between the fingers 50 along the distal region 48 of the outer shaft assembly 24 allow the flushing fluid injected between the outer shaft assembly 24 and the inner shaft assembly 22 to exit the outer shaft assembly while keeping a distal end of the outer shaft constrained by the silicone sleeve 52. The arrangement between the fingers and slits allows the larger diameter of the outer sheath 38 to take on a conical geometry when the distal region 48 is attached to the smaller diameter inner tubing 26.

With reference now to Figure 13, after the guide catheter 12 has been secured, the delivery catheter 16 is inserted into the guide catheter through the hemostatic valve. The delivery catheter 16 contains the medical implant 120 and is advanced over the guidewire 82, through the guide catheter 12 until the sleeve 52 located on a distal end of the delivery catheter is in a desired location, such as the great cardiac vein. A contrast medium that is visible under fluoroscopy, such as Renographin 60, may be inserted into the guide catheter 12 to allow an operator to ensure that the proximal anchor of the implant 120 is within the coronary sinus 17.

After the entire implant 120 has been located within the coronary sinus 17, if necessary, the guide catheter 12 may be retracted, or withdrawn completely, to fully expose the section of the delivery system on which the implant 120 is mounted. Ensuring that the section containing the implant 120 extends beyond the distal tip of the guide catheter 12 will prevent the implant from being deployed inside the guide catheter rather than inside the coronary sinus. When, by using the various marker bands 28, 30, 32, 36 on the delivery catheter 16 and by orienting the delivery catheter, the implant is determined to be in its desired position, for example, with the bridge 126 of the implant 120 being adjacent to an anterior wall of the coronary sinus, the implant may be deployed.

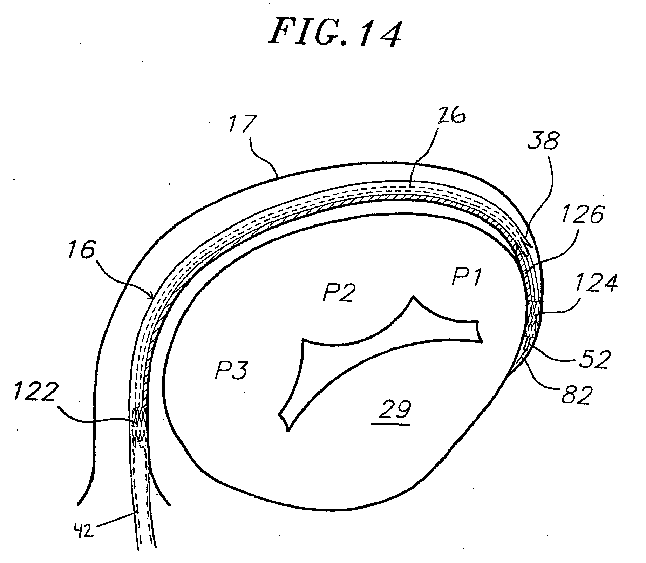

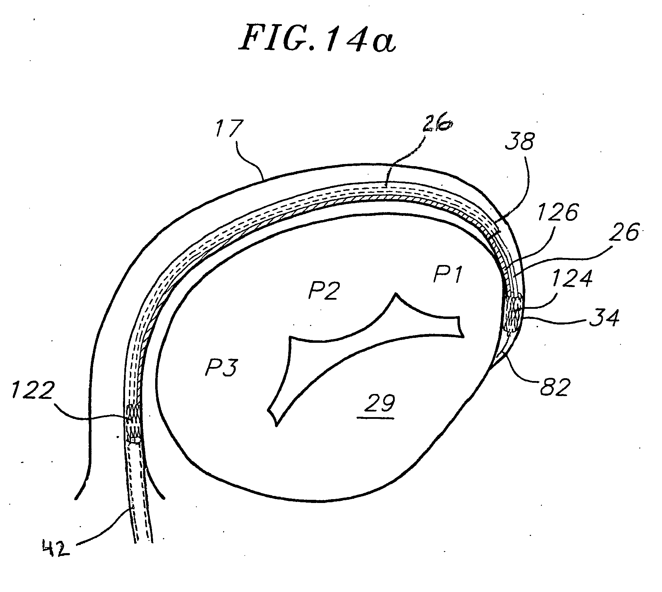

Using the sliding button 56 on the handle portion 20, an operator retracts the outer sheath 38 until the distal anchor 124 is deployed (as shown in Figure 14). As the sliding button 54 slides along the rail 58, the guiding wings 62 slide along in the groove 60 to prevent the sliding button from moving laterally. Additionally, the relative position and/or displacement of the outer sheath 38 with respect to the inner shaft assembly 22 may be determined by viewing the marker band 40 on the outer sheath and the marker bands 28, 30, 32, 36 on the inner sheath assembly 22 under fluoroscopy. The braided construction of the outer sheath 38 helps to minimize axial elongation when the outer sheath is retracted.

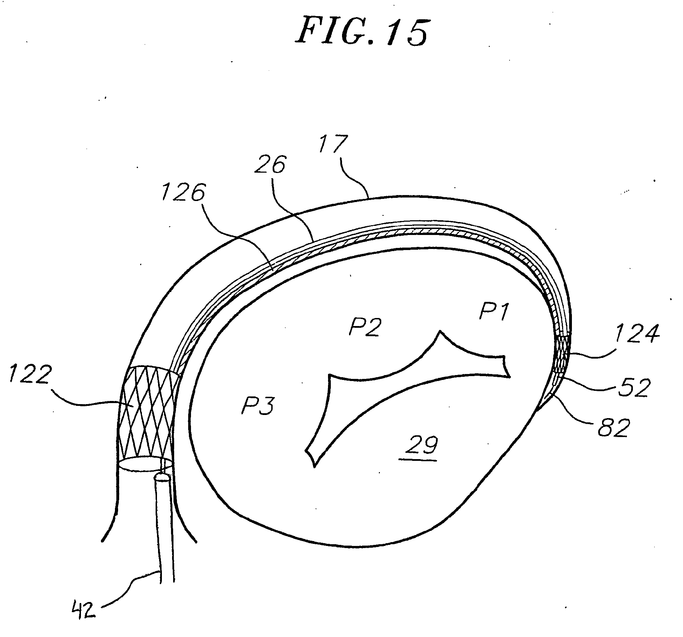

Once the distal anchor 124 is deployed, the handle portion 20 and the delivery catheter 16 may be pulled proximally to eliminate slack in the bridge. After the implant 120 has been correctly positioned, the sliding button 56 is further retracted proximally to expose the bridge 126 and the proximal anchor 121 of the implant 120 to the wall of the coronary sinus as shown in Figure 15. After both the distal and proximal anchors 122, 124 have been exposed, the delivery catheter 16 may be kept in place long enough for the anchors to completely expand. The delivery catheter is then removed from the patient. As best shown in Figure 15, the bridge 126 of the medical implant 120 is preferably located along the anterior wall of the coronary sinus 17 after deployment.

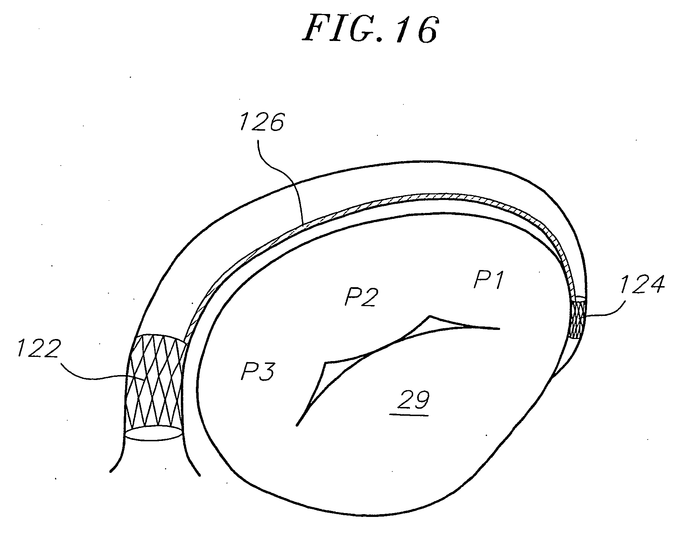

After the delivery catheter 16 has been removed from the patient, a venogram (e.g., an X-ray of a contrast medium filled vein) may be performed in the coronary sinus to ensure the patency of the implant 120. The guide catheter 12, the guide wire 82, and the introducer sheath may then be removed, leaving the implant 120 in the patient as shown in Figure 16. Over time, the implant reshapes the mitral valve annulus such that the posterior leaflet 31 is pushed toward the anterior leaflet 29, thereby closing the gap in the mitral valve.

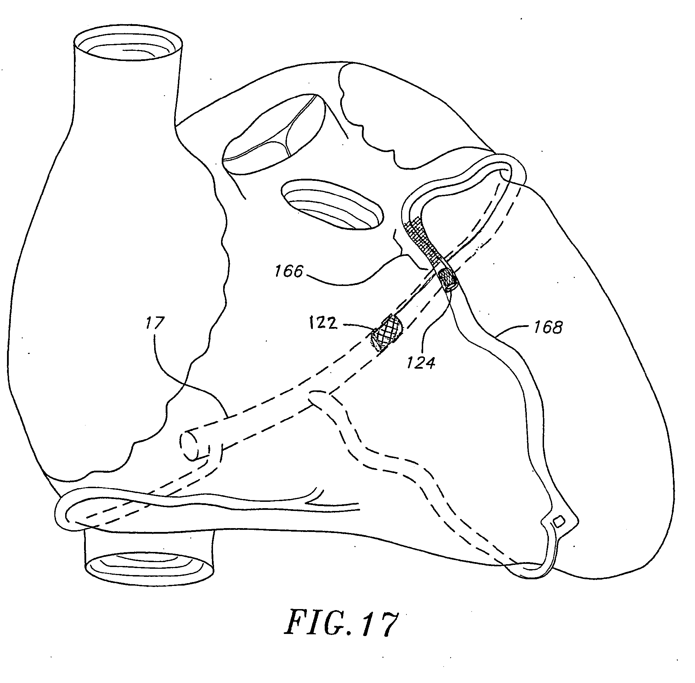

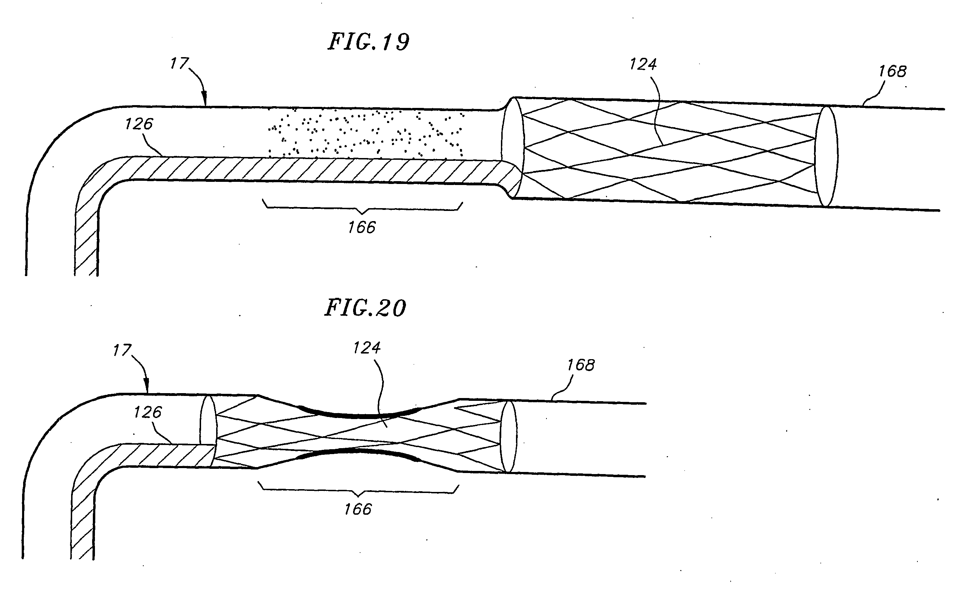

With reference now to Figure 17, in one variation, the delivery catheter is advanced deeper into the coronary sinus such that the distal anchor of the medical implant is deployed at a location distal to a fibrous region 166 of the great cardiac vein 168. This portion of the great cardiac vein is sometimes referred to as the anterior interventricular vein. As noted above, for ease of description, the great cardiac vein and anterior interventricular vein are each considered herein to be a portion of the coronary sinus 17. As schematically illustrated in Figure 19, the distal anchor 124 is deployed in the compliant region of the great cardiac vein 168 (anterior interventricular vein) that is located distal to a fibrous region 166. This anchoring point is sometimes referred to as the "Harnek fixation point." It can be seen that this anchoring point is substantially parallel to the left anterior descending coronary artery (LAD) (i.e., substantially parallel to a frontal face of the heart) rather than being located along the mitral valve annulus. As a result, using this delivery method, the distal anchor is not aligned with the proximal anchor, which may improve cinching of the mitral valve annulus as the proximal and distal anchors are pulled together. In addition, the fibrous region 166 of the vein is more resistant to dilation. The length of the region 166 is typically about 0.5 cm. By deploying the distal anchor 124 of the implant in the more compliant region of the vein (i.e., distal to the fibrous region 166), the fibrous region effectively provides a support that prevents the anchor from slipping or moving proximally. The fibrous region 166 prevents movement of the anchor because the diameter of the anchor in the expanded state is larger than the diameter of the fibrous region. In another variation, the distal anchor 124 may be expanded along the fibrous region 166 as schematically illustrated in Figure 20. In this method, proximal and distal regions of the anchor may expand to a larger diameter than a central region. This expansion causes a "waist" to be formed on the distal anchor 124, thereby allowing the distal anchor to be securely anchored in the great cardiac vein.

In combination with the delivery of the medical implant into the coronary sinus, it may be desirable to deploy one or more stents into the circumflex artery and/or left anterior descending artery (LAD) in order to ensure patency of these arteries. Stenting of the arteries is preferably performed before deploying the medical implant. The stent(s) will ensure adequate blood flow through the coronary arteries after the medical implant has adjusted to cinch and/or apply pressure to the mitral valve annulus. In one variation, it may be desirable to use a drug-eluting stent to help ensure patency. The stents may be balloon-expandable or self-expanding. In another variation, if desired, blood flow through the arteries may be improved using one or more coronary artery bypass grafts.

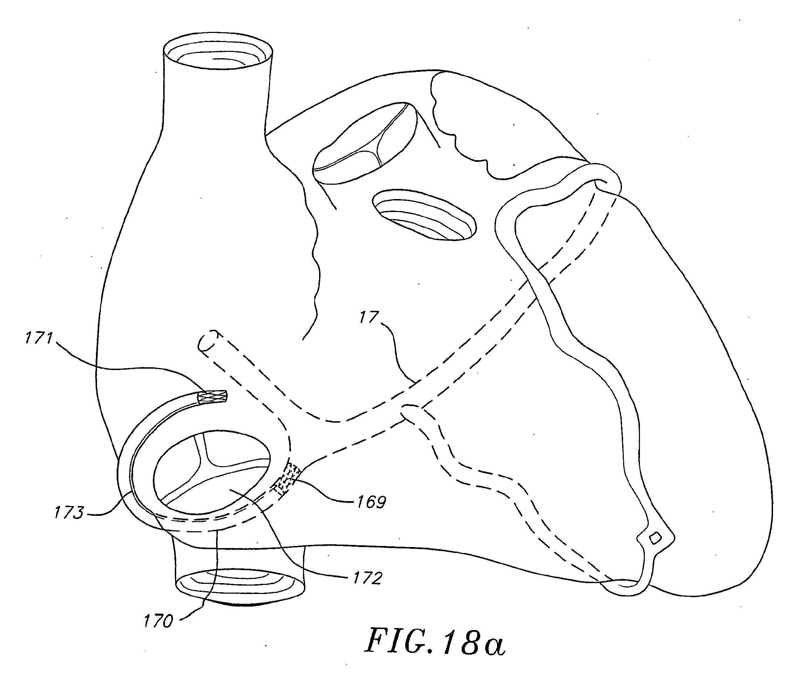

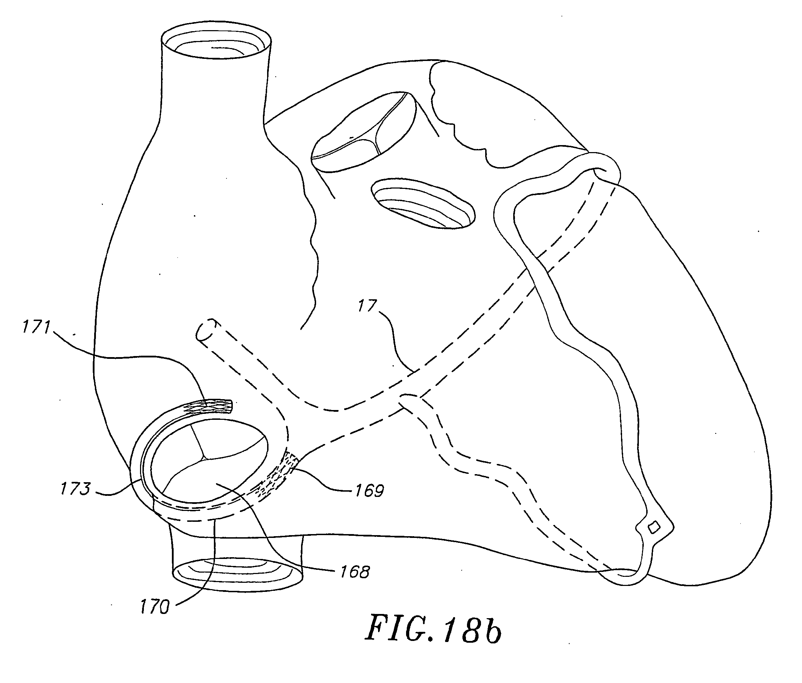

In yet another preferred method of operation, the delivery system described above is also well-suited for treating the triscupid valve of a heart. The tricuspid valve is located between the right atrium and right ventricle. Similar to mitral regurgitation, tricuspid regurgitation is typically caused by changes in the geometric configurations of the right ventricle, capillary muscles and tricuspid annulus. These geometric alterations result in incomplete leaflet coaptation during systole. With reference to Figures 18a and 18b, the delivery system may be used to advance a tricuspid repair devise (i.e., medical implant) into the small cardiac vein 170 for treating the tricuspid valve. The small cardiac vein 170 substantially encircles the tricuspid orifice and annulus and drains blood from the right ventricular myocardium to the coronary sinus 17 and finally to the right atrium. The small cardiac vein 170 is a side branch from the coronary sinus 17, taking off just inside the coronary sinus orifice and encircling the tricuspid valve 172, running parallel to the right coronary artery main stem. A tricuspid valve repair device, which may substantially resemble the medical implant 120 described above, may be loaded onto the delivery device similar to the mitral valve implant, and may be peroutaneously delivered to the treatment site. The tricuspid repair device may have a proximal anchor 169, a distal anchor 171, and a bridge 173 made with resorbable material. After the tricuspid repair device has been positioned, the resorbable material on the bridge 173 will dissolve over time, causing the bridge to foreshorten and close the gap in the tricuspid valve. As with the delivery system for the mitral valve described above, a curved distal region of the tricuspid delivery device aids in the placement of an implant in the proper orientation.

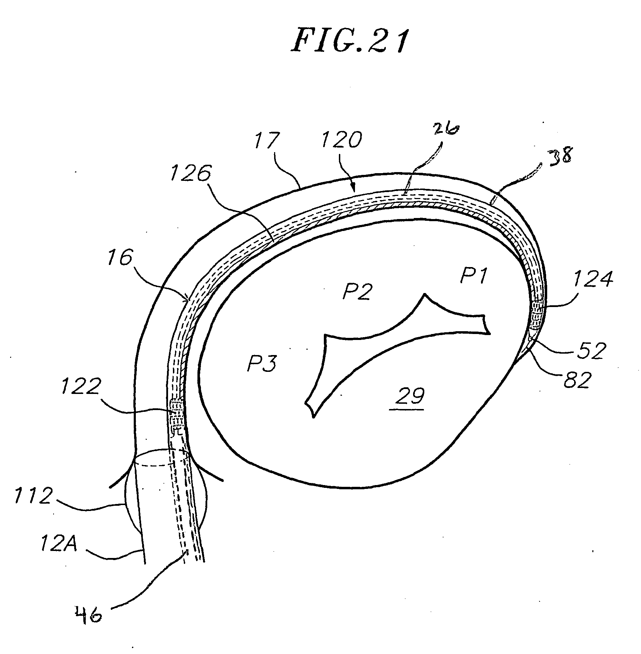

With reference to Figure 21, in yet another variation, the delivery system may be used with the guide catheter 12A for treating a mitral valve. As described above with respect to Figure 4b, the guide catheter 12A is provided with an inflatable balloon along the distal end region. As a result, the guide catheter 12A may be secured within the coronary ostium by inflating the balloon 112, thereby improving predictability during advancement of the delivery catheter. In another advantageous feature, the balloon may also be inflated to occlude the ostium for reducing or preventing leakage of radiopaque dye that may be injected into the coronary sinus 20 to enhance visualization of the vessel under fluoroscopy. In this embodiment, the balloon is preferably formed of a compliant material. Still further, the balloon may be used to dilate (i.e., expand the diameter of) the coronary sinus to facilitate advancement of the medical implant into the coronary sinus. In this application, it may be desirable to advance the distal end of the guide catheter 12A deeper into the coronary sinus before advancing the delivery catheter.

A variety of preferred embodiments have been described herein, but the invention is not limited to these embodiments. Various modifications may be made within the scope without departing from the subject matter of the invention read on the appended claims, the description of the invention, and the accompanying drawings.

## Claims

1. An apparatus for treating a mitral valve, comprising a delivery catheter (16) including an inner tubing (26), having an inflatable balloon (34) disposed along a distal end region of the inner tubing (26), and an outer sheath (38), wherein a distal portion of the inner tubing (26) is configured to be positioned within the outer sheath (38) and to provide relative displacement between the inner tubing (26) and the outer sheath (38), wherein a portion of the inflatable balloon (34) is configured to expand when the distal portion of the inner tubing (26) is positioned within the outer sheath (38), and thereby engage an inner wall of the outer sheath (38) to limit relative displacement between the inner tubing (26) and the outer sheath (38); wherein the inflatable balloon (34) comprises a proximal portion surrounded by a distal anchor (124) of a self-expanding medical implant, a central portion located distal to the distal anchor for engaging the inner wall of the outer sheath (38), and a distal portion extending from a distal tip of the outer sheath (38) shaped to provide a smooth transition between inner tubing and outer sheath (38); and a handle (20) is attached to a proximal end of the delivery catheter (16), the handle (20) including a sliding button attached to the outer sheath (38); wherein a self-expanding medical implant is located on the inner tubing (26) in a contracted condition and covered by the outer sheath (38) and wherein the siding button is retractable for withdrawing the outer sheath (38) and deploying the medical implant.

2. The apparatus of claim 1, wherein the inflatable balloon (34) has a tapered distal end portion configured to extend from the outer sheath (38) for facilitating advancement of the delivery catheter (16) through a patient's vasculature and into a coronary sinus.

3. The apparatus of claim 2, wherein the tapered distal end portion of the inflatable balloon (34) is coated with a lubricious coating.

4. The apparatus of claim 1, wherein at least a portion of the medical implant is disposed over the inflatable balloon (34) and wherein inflation of the inflatable balloon (34) assists in the deployment of the medical implant.

5. The apparatus of claim 1, wherein the inflatable balloon (34) is configured to disengage from the inner wall of the outer sheath (38), thereby allowing the outer sheath (38) to be retracted relative to the inner tubing (26).

6. The apparatus of claim 1, wherein the inflatable balloon (34) limits relative displacement between the inner tubing (26) and the outer sheath (38) by frictionally engaging the inner wall of the outer sheath (38).

7. The apparatus of claim 1, wherein the inner tubing (26) comprises a firs fluoroscopic marker (28, 29, 30, 32, 36), and the outer sheath (38) comprises a second fluoroscopic marker (40), and wherein the first fluoroscopic marker (28, 29, 30, 32, 36) and the second fluoroscopic marker (40) permit tracking of the relative displacement between the outer sheath (38) and the inner tubing (26).

## Patentansprüche

1. Vorrichtung zur Behandlung einer mitralen Klappe mit einem überbringenden Katheter (16), der ein inneres Rohr, eine Leitung oder ein Schlauchmaterial (26, im Folgenden: "Rohr") und einen aufblasbaren Ballon (34), welcher entlang eines distalen Endbereiches des inneren Rohrs (26) angeordnet ist, und einer äußeren Hülle (38), wobei ein distaler Bereich des inneren Rohrs (26) geeignet konfiguriert ist, so dass dieser in der äußeren Hülle (38) angeordnet werden kann und eine relative Verlagerung zwischen dem inneren Rohr (26) und der äußeren Hülle (38) gewährleistet ist, wobei ein Bereich des aufblasbaren Ballons (34) geeignet für eine Expansion konfiguriert ist, wenn der distale Bereich des inneren Rohrs (26) in der äußeren Hülle (38) angeordnet ist, und hierdurch eingreift oder in Wechselwirkung tritt mit einer inneren Wandung der äußeren Hülle (38) zur Begrenzung der relativen Verlagerung zwischen dem inneren Rohr (26) und der äußeren Hülle (38); wobei der aufblasbare Ballon (34) einen proximalen Bereich besitzt, der von einem distalen Anker (124) eines selbst-expandierenden medizinischen Implantats umgeben ist, einen zentralen Bereich besitzt, der distal von dem distalen Anker angeordnet ist zum Eingreifen in oder zum in Wechselwirkung treten mit der inneren Wandung der äußeren Hülle (38), und einen distalen Bereich besitzt, der sich von einer distalen Spitze der äußeren Hülle (38) erstreckt und geeignet geformt ist zur Gewährleistung eines glatten oder weichen Übergangs zwischen dem inneren Rohr und der äußeren Hülle (38); und wobei ein Handbetätigungsorgan (20) an einem proximalen Ende des überbringenden Katheters (16) befestigt ist oder werden kann, welches einen gleitenden Knopf aufweist, der an der äußeren Hülle (38) befestigt ist; wobei ein selbst-expandierendes medizinisches Implantat an oder auf dem inneren Rohr (26) in komprimiertem oder zusammengezogenem Zustand angeordnet ist und durch die äußere Hülle (38) abgedeckt ist, und wobei der gleitende Knopf zurückziehbar ist durch Zurückziehen der äußeren Hülle (38) und Ausfahren, Entfalten oder Einsetzen des medizinischen Implantats.

2. Vorrichtung nach Anspruch 1, **dadurch gekennzeichnet, dass** der aufblasbare Ballon (34) einen abgeschrägten, keilförmigen oder konischen distalen Endbereich besitzt, der geeignet konfiguriert ist, so dass sich dieser von der äußeren Hülle (38) erstreckt zur Vereinfachung oder Ermöglichung einer Vorwärtsbewegung des überbringenden Katheters (16) durch das Gefäßsystem eines Patienten und in einen koronaren Sinus.

3. Vorrichtung nach Anspruch 2, **dadurch gekennzeichnet, dass** der abgeschrägte, keilförmige oder konische distale Endbereich des aufblasbaren Ballons (34) beschichtet ist mit einer schmierenden Beschichtung.

4. Vorrichtung nach Anspruch 1, **dadurch gekennzeichnet, dass** zumindest ein Bereich des medizinischen Implantats auf dem aufblasbaren Ballon (34) angeordnet ist und dass ein Aufblasen des aufblasbaren Ballons (34) das Einsetzen des medizinischen Implantats unterstützt.

5. Vorrichtung nach Anspruch 1, **dadurch gekennzeichnet, dass** der aufblasbare Ballon (34) geeignet konfiguriert ist zum Beseitigen der Wechselwirkung mit der inneren Wandung der äußeren Hülle (38), wodurch ermöglicht wird, dass die äußere Hülle (38) relativ zu dem inneren Rohr (26) zurückgezogen wird.

6. Vorrichtung nach Anspruch 1, **dadurch gekennzeichnet, dass** der aufblasbare Ballon (34) die relative Verlagerung zwischen dem inneren Rohr (26) und der äußeren Hülle (38) durch reibende Wechselwirkung mit der inneren Wandung der äußeren Hülle (38) begrenzt.

7. Vorrichtung nach Anspruch 1, **dadurch gekennzeichnet, dass** das innere Rohr (26) ein erstes fluoroskopisches Markierungselement (28, 29, 30, 32, 36) aufweist, die äußere Hülle (38) ein zweites fluoroskopisches Markierungselement (40) aufweist und das erste fluoroskopische Markierungselement (28, 29, 30, 32, 36) und das zweite fluoroskopische Markierungselement (40) eine Nachverfolgung oder Überwachung der relativen Verlagerung zwischen der äußeren Hülle (38) und dem inneren Rohr (26) ermöglichen.

## Revendications

1. Appareil pour traiter une valvule mitrale, comprenant un cathéter de pose (16) comprenant un tube interne (26), ayant un ballonnet gonflable (34) disposé le long d'une région d'extrémité distale du tube interne (26), et une gaine externe (38), dans lequel une partie distale du tube interne (26) est configurée pour être positionnée à l'intérieur de la gaine externe (38) et pour fournir un déplacement relatif entre le tube interne (26) et la gaine externe (38), dans lequel une partie du ballonnet gonflable (34) est configurée pour s'expanser lorsque la partie distale du tube interne (26) est positionnée à l'intérieur de la gaine externe (38), et pour mettre ainsi en prise une paroi interne de la gaine externe (38) afin de limiter le déplacement relatif entre le tube interne (26) et la gaine externe (38) ; dans lequel le ballonnet gonflable (34) comprend une partie proximale entourée par un ancrage distal (124) d'un implant médical auto-expansible, une partie centrale positionnée de manière distale par rapport à l'ancrage distal pour mettre en prise la paroi interne de la gaine externe (38) et une partie distale s'étendant à partir d'une pointe distale de la gaine externe (38) formée pour fournir une transition régulière entre le tube interne et la gaine externe (38) ; et une poignée (20) est fixée sur une extrémité proximale du cathéter de pose (16), la poignée (20) comprenant un bouton coulissant fixé sur la gaine externe (38) ; dans lequel un implant médical auto-expansible est positionné sur le tube interne (26) dans une condition contractée et recouvert par la gaine externe (38) et dans lequel le bouton coulissant est rétractable pour retirer la gaine externe (38) et déployer l'implant médical.

2. Appareil selon la revendication 1, dans lequel le ballonnet gonflable (34) a une partie d'extrémité distale progressivement rétrécie configurée pour s'étendre à partir de la gaine externe (38) afin de faciliter la progression du cathéter de pose (16) à travers le système vasculaire d'un patient et dans un sinus coronarien.

3. Appareil selon la revendication 2, dans lequel la partie d'extrémité distale progressivement rétrécie du ballonnet gonflable (34) est recouverte avec un revêtement lubrifiant.

4. Appareil selon la revendication 1, dans lequel au moins une partie de l'implant médical est disposée sur le ballonnet gonflable (34) et dans lequel le gonflage du ballonnet gonflable (34) aide à déployer l'implant médical.

5. Appareil selon la revendication 1, dans lequel le ballonnet gonflable (34) est configuré pour se dégager de la paroi interne de la gaine externe (38), permettant ainsi de rétracter la gaine externe (38) par rapport au tube interne (26).

6. Appareil selon la revendication 1, dans lequel le ballonnet gonflable (34) limite le déplacement relatif entre le tube interne (26) et la gaine externe (38) en mettant en prise par frottement la paroi interne de la gaine externe (38).

7. Appareil selon la revendication 1, dans lequel le tube interne (26) comprend un premier marqueur fluoroscopique (28, 29, 30, 32, 36) et la gaine externe (38) comprend un deuxième marqueur fluoroscopique (40) et dans lequel le premier marqueur fluoroscopique (28, 29, 30, 32, 36) et le deuxième marqueur fluoroscopique (40) permettent de suivre le déplacement relatif entre la gaine externe (38) et le tube interne (26).
